(19) Europäisches Patentamt
European Patent Office
Office européen des brevets

(11) Veröffentlichungsnummer : **0 395 588 B1**

# (12) EUROPÄISCHE PATENTSCHRIFT

(45) Veröffentlichungstag der Patentschrift :
**08.06.94 Patentblatt 94/23**

(51) Int. Cl.$^5$ : **C07D 307/79,** C07D 307/82, D06L 3/12, D21H 21/30, C11D 3/42

(21) Anmeldenummer : **90810314.6**

(22) Anmeldetag : **19.04.90**

## (54) Dibenzofuranylbiphenyle.

(30) Priorität : **28.04.89 CH 1629/89**

(43) Veröffentlichungstag der Anmeldung :
**31.10.90 Patentblatt 90/44**

(45) Bekanntmachung des Hinweises auf die Patenterteilung :
**08.06.94 Patentblatt 94/23**

(84) Benannte Vertragsstaaten :
**CH DE ES FR GB IT LI**

(56) Entgegenhaltungen :
**EP-A- 0 344 776**
**DE-A- 2 843 850**
**US-A- 4 002 423**

(73) Patentinhaber : **CIBA-GEIGY AG**
**Klybeckstrasse 141**
**CH-4002 Basel (CH)**

(72) Erfinder : **Weber, Kurt, Dr.**
**Rennweg 98**
**CH-4052 Basel (CH)**
Erfinder : **Eckhardt, Claude, Dr.**
**16, Rue des Jonquilles**
**F-68400 Riedisheim (FR)**
Erfinder : **Meyer, Hans Rudolf**
**Bollwerkstrase 102**
**CH-4102 Binningen (CH)**

EP 0 395 588 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft gezielt sulfonierte Dibenzofuranylbiphenylverbindungen, deren Herstellung und Verwendung als optische Aufheller zum Aufhellen von Textilmaterialien oder Papier oder deren Einsatz in Persäure enthaltenden Waschmitteln.

Gemische von sulfonierten Benzofuranylbiphenylverbindungen mit undefinierter Zusammensetzung und Struktur sowie deren Verwendung als optische Aufheller sind lange bekannt (DE-A-22 38 734, DE-A-22 38 628, DE-A-23 61 338 und DE-A-28 43 850). Jedoch war es bislang nicht möglich Einzelverbindungen derartiger Gemische mit einheitlichen Strukturen herzustellen.

Es hat sich nun überraschenderweise gezeigt, dass sulfonierte und strukturell definierte Dibenzofuranylbiphenylverbindungen nach bestimmten Verfahren gezielt herstellbar sind.

Gegenstand der Erfindung sind somit neue Dibenzofuranylbiphenylverbindungen der Formel (I)

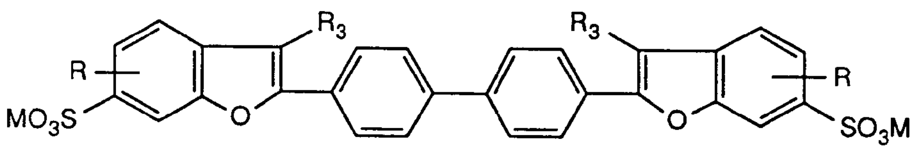

(I)

die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert sind und worin $R_3$ = $C_1$-$C_4$-Alkyl, Halogen oder Phenyl, M = Wasserstoff und/oder ein Aequivalent eines nicht-chromophoren Kations bedeuten.

Als Halogene kommen vor allem Fluor, Chlor und Brom in Frage, insbesondere jedoch Chlor.

Als $C_1$-$C_4$-Alkylreste (bzw. $C_1$-$C_4$-Alkoxyreste) kommen unverzweigte oder verzweigte Alkyl- (bzw. Alkoxy-) reste in Betracht. Diese Alkyl- (bzw. Alkoxy-) reste können ihrerseits substituiert sein mit z.B. Aryl- (Phenyl-, Tolyl-), $C_1$-$C_4$-Alkyl-, $C_1$-$C_4$-Alkoxy-, OH- oder CN-Gruppen. Die Phenylreste können anderseits mit Alkyl, Alkoxy oder Halogen substituiert sein.

M in der Bedeutung eines nicht-chromophoren Kations steht beispielsweise für Erdalkalimetall wie Magnesium und Calcium, vorzugsweise jedoch für Alkalimetall wie Lithium, Natrium, Kalium sowie gegebenenfalls substituiertes Ammonium wie Ammonium, Mono-, Di- oder Triethanolammonium, Mono-, Di- oder Tripropanolammonium oder Mono-, Di-, Tri- oder Tetramethylammonium.

Bevorzugt sind Verbindungen der Formel (II)

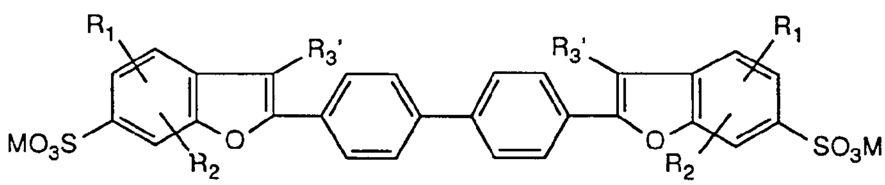

(II)

worin $R_1$ = Wasserstoff, $C_1$-$C_4$-Alkyl, Chlor, $C_1$-$C_4$-Alkoxy $R_2$ = Wasserstoff, $C_1$-$C_4$-Alkyl, Chlor oder $C_1$-$C_4$-Alkoxy, $R_3'$ = $C_1$-$C_4$-Alkyl, Chlor, Phenyl, M = Wasserstoff und/oder ein Aequivalent eines nicht chromophoren Kations bedeuten.

Von besonderem Interesse sind jedoch Verbindungen der Formel (III)

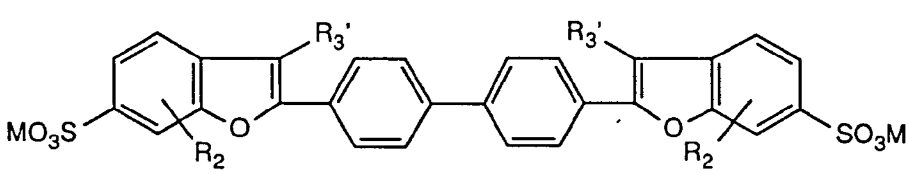

(III)

worin $R_2$, $R_3'$ und M die oben angegebene Bedeutung haben. $R_2$ ist vorzugsweise Wasserstoff, 5-Methyl, 5-Ethyl oder 5-Chlor und $R_3'$ ist vorzugsweise Methyl, Ethyl oder Phenyl.

Ein weiterer Gegenstand dieser Erfindung sind Verfahren zur Herstellung der Verbindungen der Formel (I), die dadurch gekennzeichnet sind, dass man ein Mol der Verbindung der Formel (X)

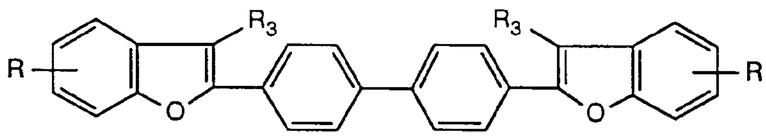 (X)

die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert ist und worin $R_3$ = $C_1$-$C_4$-Alkyl, Halogen oder Phenyl ist, mit

a) mindestens stöchiometrischen Mengen eines $SO_3$/Basen-Komplexes, in einem inerten organischen Lösungsmittel, bei Temperaturen von 20°C bis zum Siedepunkt des verwendeten Lösungsmittels umsetzt oder

b) die Verbindung der Formel (X) mit Chlorsulfonsäure bei 0-80°C oder

c) die Verbindung der Formel (X) mit Eisessig und konzentrierter Schwefelsäure oder Oleum bei Temperaturen von 80° bis 140°C

behandelt und die erhaltenen Sulfosäuren gegebenenfalls mit geeigneten Basen, wie wässrigem Alkali, Ammoniak oder Aminen neutralisiert. Gegebenenfalls als Nebenprodukt gebildete Sulfochloride werden vorangehend oder gleichzeitig mit Wasser in der Hitze verseift.

Die Verbindungen der Formel (X) sind teilweise bekannt und können nach bekannter Art und Weise hergestellt werden.

Die neuen Ausgangsverbindungen der Formel (X) mit $R_3$ = Cl erhalt man durch Chlorierung von Verbindungen der Formel (X),worin $R_3$ = H bedeutet, mit Phosphorpentachlorid bei 100 bis 200° in geeigneten Lösungsmitteln wie Chlorbenzol oder Dichlorbenzol

Unter $SO_3$/Basen-Komplex sind Additionsverbindungen von $SO_3$ an organische Basen, z.B. Dioxan, vorzugsweise stickstoffhaltige Basen, wie zum Beispiel Triethylamin, N-Ethyldiisopropylamin, Dimethylformamid (DMF) und Pyridin zu verstehen. Die Stabilität dieser Additionsverbindungen ist dabei entscheidend für den Sulfonierungsgrad.

So werden 2 bis 6, insbesondere 3 bis 5 Mol $SO_3$/Pyridin-Komplex (bezogen auf den $SO_3$-Gehalt) pro Mol der Verbindung der Formel (X) oder 2 bis 6, insbesondere 3 bis 5 Mol $SO_3$/DMF-Komplex (bezogen auf den $SO_3$-Gehalt) pro Mol der Verbindung der Formel (X) verwendet. $SO_3$/Basen-Komplexe sind bekannt und können nach bekannten Methoden hergestellt werden (E.E. Gilbert, E.P. Jones, Ind. Enging. Chem. 49, Nr. 9, Teil II, S. 1553 ff (1957); Beilstein 20, III/IV, 2232).

Bei Verfahren b) wird vor allem ein Mol der Verbindung der Formel (X) mit 2 bis 20, insbesondere 2 bis 4 Mol Chlorsulfonsäure bei Temperaturen von 0°C bis 80°C, insbesondere 5°C bis 40°C ohne oder vorzugsweise in einem inerten organischen Lösungsmittel umgesetzt.

Inerte organische Lösungsmittel sind beispielsweise gesättigte aliphatische Kohlenwasserstoffe wie Gasolin, Petrolether und Ligroin, halogenierte, aliphatische Kohlenwasserstoffe wie Chloroform, Tetrachlorkohlenstoff, Dichlorethan, Trichlorethan, Tetrachlorethan, Dichlorpropan, Trichlorpropan, Dichlordifluormethan und Dichlortetrafluorethan, Chlorbenzole wie Mono-, Di- und Trichlorbenzol, Nitrobenzole wie Nitrobenzol und Nitrotoluol sowie mono- oder dicyclische Kohlenwasserstoffe wie Cyclohexan, Methylcyclohexan und Dekalin.

Verbindungen der Formel (I) finden Verwendung zur Aufhellung von Textilmaterialien, wobei Polyamide, Wolle und Baumwolle besonders hervorzuheben sind und von Papier.

Begründet durch den gestiegenen Anteil an Kunstfasern bzw. Mischfasern in den heute hergestellten Textilien, dem Wunsch, bunte Wäsche nicht mehr separat zu waschen sowie aus Energie-Sparmassnahmen wird in vielen Ländern die Wäsche nicht mehr bei 90°C-95°C oder beim Kochen sondern bei tieferen Temperaturen gewaschen. Dies führte dazu, dass die bislang meistens in Waschmitteln enthaltenen, als Bleichmittel wirkenden, Perborate durch Hilfsmittel wie z.B. Tetraacetylethylendiamin (TAED) aktiviert werden mussten, um akzeptable Bleicheffekte auch bei Waschtemperaturen von 60-80°C zu erlangen. Bei noch tieferen Waschtemperaturen liefern auch die Perborat-AktivatorSysteme keine befriedigenden Ergebnisse mehr.

Seit einiger zeit werden daher Waschmittel beschrieben (DE-OS-27 56 583, EP-A-145 438, GB-2 141 754, GB-2 141 755, US-4 028 263, GB-59 272), die stärkere Bleichmittel wie z.B. Persäuren enthalten. Diese neuen Bleichmittel zeigen einerseits zwar hervorragende Bleicheffekte schon bei Temperaturen ab 20°C, andererseits zerstören sie aber die üblichen in Waschmitteln enthaltenen optischen Aufheller.

Oben beschriebene spezielle Dibenzofuranyl-Verbindungen weisen eine ausgezeichnete Stabilität in Waschmitteln auf, die solche starken Bleichmittel enthalten. Unter durchschnittlichen Lagerbedingungen und sogar unter verschärften Bedingungen (Temperaturen über 30°C und Luftfeuchtigkeit über 60 %) sind diese speziellen Dibenzofuranyl-Aufheller im Waschmittel über mehrere Monate vollständig stabil oder werden höchstens in einem für die Praxis nicht störenden Mass abgebaut.

Gegenstand der Erfindung sind somit ferner lagerstabile Waschmittel enthaltend 0,5 bis 30 % einer anorganischen oder organischen Persäure oder deren Salze oder Mischungen von Persäuren oder deren Salze sowie 0,03 % bis 0,8 % eines optischen Aufhellers oder eine Mischung von optischen Aufhellern, dadurch gekennzeichnet, dass die optischen Aufheller Dibenzofuranylbiphenylverbindungen der Formel (I)

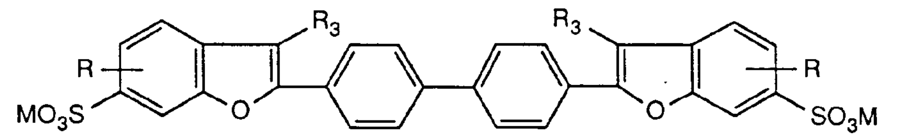

(I)

sind, die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert sind und worin $R_3$ = $C_1$-$C_4$-Alkyl, Halogen oder Phenyl, M = Wasserstoff und/oder ein Aequivalent eines nicht-chromophoren Kations bedeuten.

Bevorzugt sind Verbindungen der Formel (II)

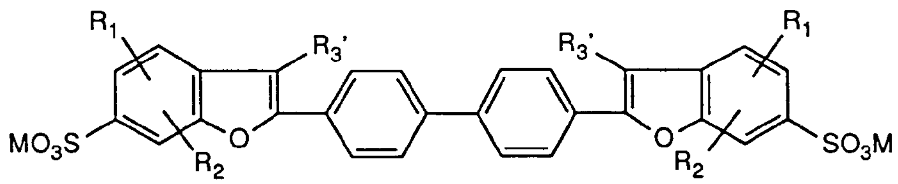

(II)

worin
$R_1$ = Wasserstoff, $C_1$-$C_4$-Alkyl, Chlor, $C_1$-$C_4$-Alkoxy $R_2$ = Wasserstoff, $C_1$-$C_4$-Alkyl, Chlor oder $C_1$-$C_4$-Alkoxy, $R_3'$ = $C_1$-$C_4$-Alkyl, Chlor oder Phenyl, M = Wasserstoff und/oder ein Aequivalent eines nicht chromophoren Kations bedeuten.

Von besonderem Interesse sind jedoch Verbindungen der Formel (III)

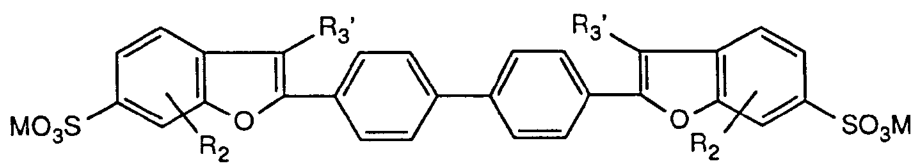

(III)

worin $R_2$, $R_3'$ und M die oben angegebene Bedeutung haben. $R_2$ ist vorzugsweise Wasserstoff, 5-Methyl, 5-Ethyl oder 5-Chlor und $R_3'$ ist vorzugsweise Methyl, Ethyl oder Phenyl.

Bei den Persäuren bzw. deren Salze handelt es sich um in der Literatur beschriebene oder auf dem Markt befindliche organische oder anorganische Verbindungen die Textilien schon bei Temperaturen ab 20°C bleichen. Insbesondere sind die organischen Persäuren wie z.B. Mono- oder Polypersäuren mit Alkyl-Ketten von mindestens 3, vorzugsweise 6 bis 20 Kohlenstoffatomen vor allem jedoch Diperoxydicarbonsäuren mit 6 bis 12 C-Atomen, wie Diperoxyperazelainsäure, Diperoxypersebacinsäure, Diperoxyphthalsäure und/oder Diperoxydodecandisäure (DPDDA) von Interesse. Es können aber auch sehr wirksame, anorganische Persäuren wie Persulfat und/oder Percarbonat eingesetzt werden. Die einzusetzende Menge an organischen Persäuren liegt vorzugsweise bei 0,5 % bis 10 %, insbesondere 1 % bis 5 % und an anorganischen Persäuren vorzugsweise bei 1 % bis 30 %, insbesondere 10 % bis 20 %, bezogen auf das gesamte Waschmittelgewicht und gegebenenfalls in Kombination mit kleinen Mengen an Verbindungen, die die Bleichwirkung der Persäuren verstärken wie katalytisch wirkender bivalenter Metall-Salze, wie sie in der US 4 655 782 und US 4 655 953 beschrieben werden. Bevorzugt werden Metallsalze von Kupfer und/oder Mangan verwendet.

Selbstverständlich können auch Mischungen von organischen und/oder anorganischen Persäuren bzw. -Salzen eingesetzt werden.

Die Zugabe der Persäuren in das Waschmittel erfolgt vor allem durch Mischen der Komponenten z.B. mit Hilfe von Schnecken-Dosier-Systemen und/oder Wirbelschicht-Mischern.

Bei den Waschmitteln handelt es sich um trockene Waschmittel üblicher Zusammensetzungen. In der Regel enthalten sie neben der erfindungsgemässen Kombination aus Persäure und Aufheller, beispielsweise anionische, nicht-ionogene, amphotere und/oder kationische Tenside, Builder wie z.B. Pentanatriumtripolyphosphat oder Ersatzprodukte wie Phosphonate, Polycarboxylate, Acryl-Malein-Copolymere, Zeolithe,

Nitrilotriacetat, Ethylendiaminotetraessigsäure, Schmutzsuspendiermittel wie z.B.Natriumcarboxymethylcellulose, Salze zur Einstellung des pH-Werts wie z.B. Alkali- oder Erdalkalisilikate, Schaum-Regulatoren wie z.B. Seife, Salze zur Einstellung der Sprühtrocknungs- und Granulat-Eigenschaften wie z.B. Natriumsulfat, Parfums, sowie gegebenenfalls antistatische und weichmachende Mittel, Enzyme, Photobleichmittel, Pigmente und/oder Nuancierungsmittel. Selbstverständlich sollen diese Bestandteile gegenüber dem eingesetzten Bleichsystem stabil sein.

Dank der erfindungsgemässen Kombination ist es möglich, Waschmittel anzubieten, die dem üblichen Standard wie z.B. punkto Waschkraft, Fleckenentfernung, Erfrischung des Aussehens der gewaschenen Artikel entsprechen, auch wenn bei Temperaturen von 20°C-60°C gewaschen wird. Vorteilhafterweise können somit Buntwäsche und Weisswäsche unabhängig von der Faser zusammen gewaschen werden.

Die folgenden Beispiele dienen zur Erläuterung der Erfindung; Teile bedeuten Gewichtsteile und Prozente Gewichtsprozente.

Beispiel 1: 6,5 ml Oleum 65 % werden in 200 ml Eisessig vorgelegt. Dann werden unter Rühren 13,7 g der Verbindung der Formel

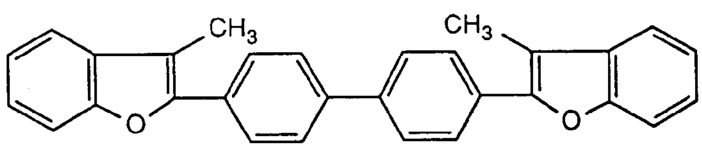

(101)

eingetragen, auf 100-105°C erhitzt und bei dieser Temperatur während 1 Stunde gerührt. Nach dem Abkühlen auf 60°C werden 25 g Natriumacetat in 40 ml Wasser zugegeben, auf Raumtemperatur abkühlen gelassen und das auskristallisierte Produkt abgenutscht. Nach dem Umkristallisieren aus einer Mischung von 800 ml Wasser und 400 ml Methylcellosolve und einer zweiten Umkristallisation aus einer Mischung von 500 ml Wasser und 500 ml Methylcellosolve erhält man 12 g der Verbindung der Formel

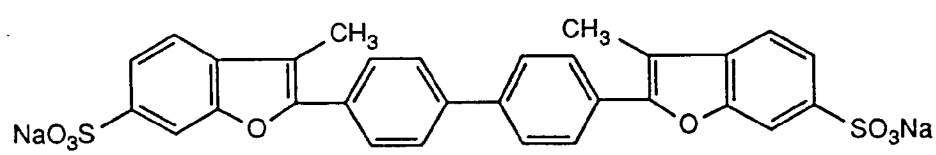

(102)

als blassgelbes Kristallpulver. UV-Absorption: λmax = 351nm; ε= 74450 (in DMF/$H_2O$ 1:1)

In analoger Weise erhält man die Verbindung der Formel

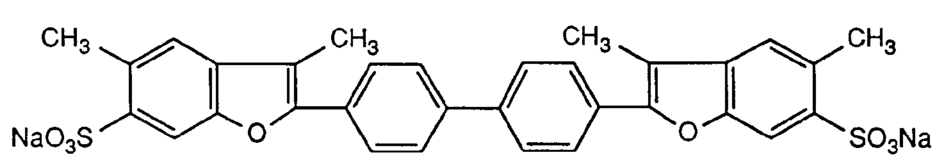

(103)

blassgelbes Kristallpulver

UV-Absorptionsspektrum: $\lambda_{max}$: 353 nm; ε = 76300 (DMF-$H_2O$ 1:1)

Die Verbindung der Formel (101) kann gemäss DAS 22 38 628 erhalten werden.

Beispiel 2: 62,1 g der Verbindung der Formel (101) und 50 g Schwefeltrioxid-dimethylformamidkomplex werden in 370 ml Nitrobenzol unter Rühren vorgelegt, auf 115°C erhitzt und 2 Stunden bei dieser Temperatur gerührt. Nach dem Abkühlen auf 60°C wird mit 10%iger Natronlauge auf pH9 gestellt, das Nitrobenzol mittels Wasserdampf-Destillation entfernt, die wässrige Suspension auf 10°C abgekühlt, abgenutscht, mit einer 5%igen Kochsalzlösung gewaschen und unter Vakuum bei 100°C getrocknet. Man erhält 86,3 g blassgelbes Produkt, welches 94,6 % der Verbindung (102) enthält.

Beispiel 3: 18,5 ml Oleum 65 % werden in 450 ml Eisessig vorgelegt. Dann werden unter Rühren 44,2 g der Verbindung der Formel

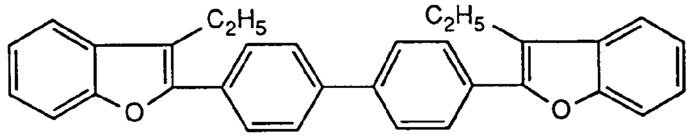

(104)

Schmelzpunkt: 190-191°C

eingetragen, auf 100°C erhitzt und während 1 Stunde bei dieser Temperatur gerührt. Nach dem Abkühlen auf Raumtemperatur wird die Reaktionsmischung auf eine Lösung von 100 g Natriumacetat in 450 ml Wasser gegossen, auf 100°C erhitzt, mit 7 g Aktivkohle versetzt und heiss filtriert. Nach dem Abkühlen auf Raumtemperatur wird das auskristallisierte Produkt abgenutscht und unter Vakuum getrocknet. Das so erhaltene Produkt wird zweimal aus einer Mischung von 150 ml Ethanol und 150 ml Wasser unter Zusatz von Aktivkohle umkristallisiert. Man erhält 45 g der Verbindung der Formel

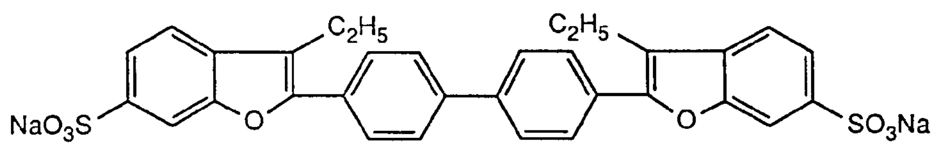

(105)

blassgelbes Kristallpulver

UV-Absorptionsspektrum: $\lambda_{max}$: 350nm; $\varepsilon$ = 69864 (DMF/$H_2O$ 1:1)

Die Verbindung der Formel (104) kann analog der Verbindung der Formel (101) erhalten werden.

Beispiel 4: 19,4 g der Verbindung der Formel

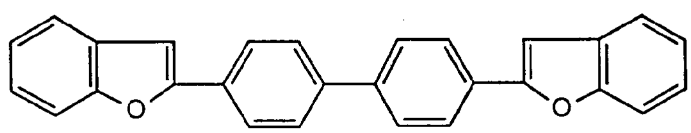

(106)

und 25,0 g Phosphorpentachlorid werden in 150 ml Chlorbenzol verrührt und die Suspension auf Rückflusstemperatur erhitzt. Nachdem das Ausgangsprodukt sich vollständig gelöst hat und die Salzsäuregasentwicklung beendet is (ca. 1 h) lässt man abkühlen und versetzt vorsichtig mit 50 ml Methanol. Das ausgefallene Produkt wird abgenutscht, mit Methanol gewaschen und im Vakuum getrocknet. Man erhält 19,6 g der Verbindung der Formel

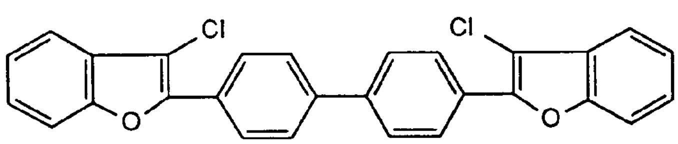

(107)

in Form fast farbloser Kristalle. Smp. 210-11° (umkristallisiert aus Xylol).

Durch Sulfierung mit Chlorsulfonsäure in Tetrachlorethan gemäss Beispiel 6 erhält man die Verbindung der Formel

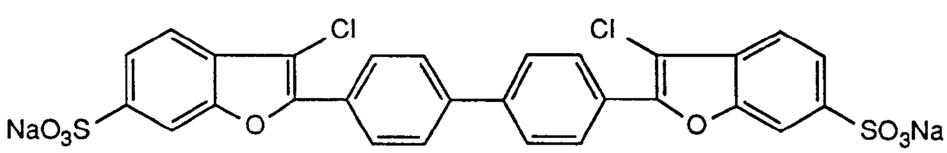

(108).

Beispiel 5: Zu einer Suspension von 4,6 g der Verbindung der Formel (107) und 4,8 g Thionylchlorid in 30 ml Tetrachlorethan tropft man unter Rühren bei 90°C im Verlauf 1/4 Stunde eine Lösung von 1,4 ml Chlorsulfonsäure in 20 ml Tetrachlorethan. Nach beendeter Reaktion (90-120°C) lässt man abkühlen, nutscht das ausgefallene Produkt ab und trocknet es im Vakuum bei 90°C. Man erhält 5,3 g des Disulfochlorids [Smp. 297°

(Zers.) umkrist. aus Chlorbenzol]. 3,3 g Disulfochlorid werden in 18 ml Pyridin und 2 ml Wasser 20 Min. bei Rückflusstemperatur erhitzt. Die Lösung wird im Vakuum zur Trockne eingedampft und der Rückstand aus n-Propanol-Wasser 7:3 bis 9:1 umkristallisiert unter Zuhilfenahme von Aktivkohle zur Klärfiltration. Man erhält 2,4 g der Verbindung der Formel

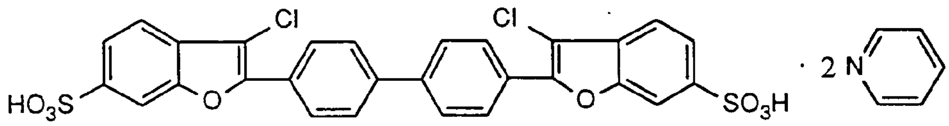

(109)

Beispiel 6: Zu einer feinen Dispersion von 7,7 g der Verbindung der Formel

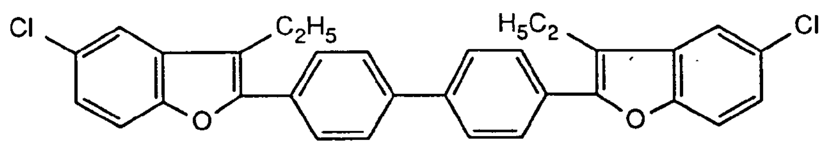

(110)

in 100 ml Tetrachlorethan tropft man unter Rühren bei 5°C im Verlauf 1/2 Stunde 5,24 g Chlorsulfonsäure. Man lässt noch 2 Stunden bei Raumtemperatur nachrühren, nutscht das dunkle Produkt ab, wäscht mit Tetrachlorethan und suspendiert es in 150 ml Wasser. Das restliche Tetrachlorethan wird in der Hitze mit Wasser azeotrop abdestilliert unter gleichzeitiger Neutralisation mit Natronlauge (30 %), bis sich ein konstanter pH von 9 einstellt. Man dampft im Vakuum am Rotationsverdampfer zur Trockne ein, löst den Rückstand in einer Mischung von n-Propanol-Wasser 3:2, fügt n-Propanol hinzu bis zu einem Verhältnis von 2:1, filtriert heiss mit Aktivkohle und engt das Filtrat ein unter weiterem Zufügen von n-Propanol bis das Produkt auskristallisiert. Dieses wird bei 0°C abgenutscht, mit n-Propanol gewaschen und im Vakuum getrocknet. Man erhält 8,8 g der Verbindung der Formel

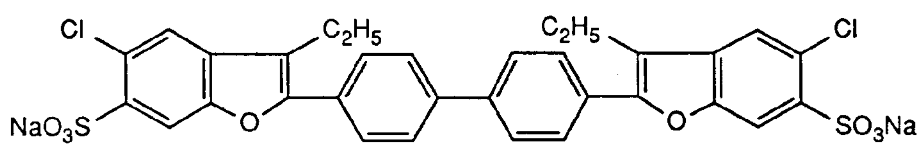

(111)

die noch 1 Mol Kristallwasser enthält.

Die als Ausgangsprodukt verwendete Verbindung der Formel (110) wird wie folgt hergestellt:

Zu einer Lösung von 36,9 g 5-Chlor-2-hydroxy-propiophenon in 100 ml DMF tropft man unter Rühren bei Raumtemperatur 36 g einer 30%igen Natriummethylat-Lösung. Nach 2 Stunden fügt man 26 g 4,4'-Bis-chlormethyl-biphenyl hinzu und erhitzt 3 Stunden bei 100°, wobei etwa Methanol abdestilliert. Man kühlt auf 5°C ab, nutscht das ausgefallene Produkt ab, wäscht mit Ethanol und Wasser und trocknet es im Vakuum bei 100°C. Man erhält 47,6 g der Verbindung der Formel

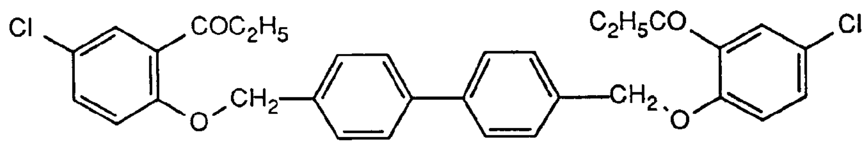

(112)

Smp. 167-8° (Toluol).

Zu einer Lösung von 27,4 g der Verbindung der Formel (112) in 70 ml Dimethylsulfoxid fügt man bei 130°C im Verlauf 1/2 Stunde portionenweise 2,7 g Natriummethylat. Nach 2 Stunden lässt man auf Raumtemperatur abkühlen, verdünnt mit 60 ml Methanol, nutscht das ausgefallene Produkt ab, wäscht mit Methanol und Wasser und trocknet es im Vakuum bei 100°. Man erhält 22,8 g der Verbindung der Formel (110); Smp. 233-5°C (Xylol).

Beispiel 7: In der vorangehend beschriebenen Weise werden aus den entsprechenden Komponenten die folgenden Bis-phenoläther ($A_1$), Dibenzofurane ($A_2$) und Dibenzofuran-disulfosäuren ($A_3$) hergestellt:

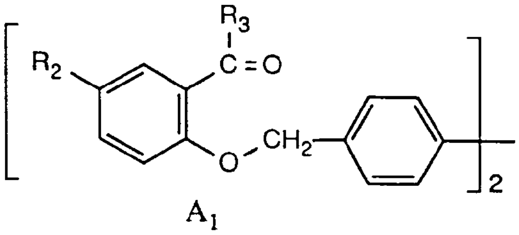

$A_1$

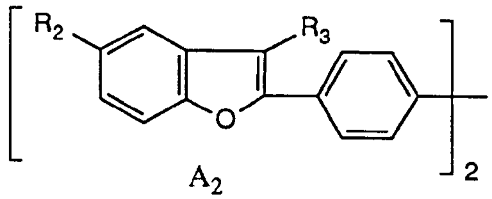

$A_2$

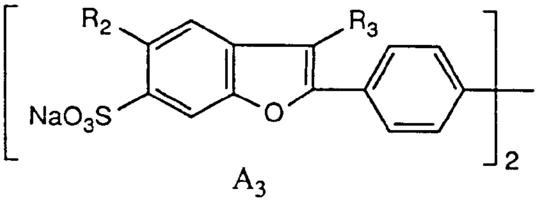

$A_3$

| $R_2$ | $R_3$ | ($A_1$) Smp. (°C) | ($A_2$) Smp. (°C) | ($A_3$) Smp. (°C) | No. |
|---|---|---|---|---|---|
| Cl | $CH_3$ | 159-60 | 232-4 | >300 | (113) |
| $CH_3$ | $C_2H_5$ | 166-68 | 214-5 | >300 | (114) |
| $OCH_3$ | $CH_3$ | 145-46 | 240-2 | >300 | (115) [1] |
| H | $C_6H_5$ | 170-71 | 264-6 | >300 | (116) [2] |
| $CH_3$ | $C_6H_5$ | 208-10 | 256-7 | >300 | (117) [2] |

[1] Um das besser lösliche Ammoniumsalz zu erhalten, wird anstatt mit Natronlauge mit Ammoniak neutralisiert, nach Aufkochen und Abdestillieren des restlichen Tetrachlorethans.

[2] Sulfierung mit $SO_3$-Dimethylformamid-Komplex (20 % Überschuss) in Nitrobenzol bei 120°C über Nacht.

Beispiel 8: Ein Stück Polyamid 66 (Nylon Webtrikot) wird bei einem Flottenverhältnis von 1:20 in weichem Wasser, das 0,1% der Verbindung der Formel (102) (Gewichtsprozent bezogen auf das Textilmaterial), sowie 3 g/l stabilisiertes Hydrogensulfit und 1 ml/l Essigsäure 80%ig enthält, innerhalb von 30 Minuten von 40°C auf 98°C aufgeheizt, dann während 30 Minuten bei 98°C behandelt, und innerhalb von 15 Minuten wieder auf 40°C abgekühlt, kalt gespült und in einem Trockenschrank bei 60°C getrocknet. Es resultiert ein hoher, brillanter Weissgrad. Nach üblicher Standardmethode in einem ®Xenotest-Apparat belichtet, zeigt dieser Weisseffekt eine sehr gute Lichtechtheit.

Beispiel 9: Es wird analog wie in Beispiel 8 vorgegangen, jedoch wird anstelle der Verbindung (102) die Verbindung (103), oder die Verbindung (108), oder die Verbindung (116) verwendet.

Auch hier resultieren hohe, brillante Weisseffekte mit sehr guten bis ausgezeichneten Lichtechtheiten.

Beispiel 10: Durch Sprühtrocknung eines Slurry bestehend aus 1 Teil Wasser und 1 Teil eines Waschmittels folgender Zusammensetzung:

8,4 g lineares Dodecylbenzolsulfonat,
3,1 g Talgalkohol-tetradecan-ethylenglykolether (14 EO),
3,7 g Na-Seife (vorwiegend aus Behen-Säure und $C_{14}$-$C_{20}$-Säuren),

45,8 g Na-Tripolyphosphat,
7,9 g Na.Silikat,
2,0g Mg-silikat,
1,2 g Carboxymethylcellulose,
0,2 g Ethylendiamintetraacetat,
22,2 g Na-Sulfat und
0,1 g der Verbindung (102)
wird ein Waschmittelgranulat mit einer Restfeuchte von ca. 5 % hergestellt.

4 g dieses Waschmittels werden in 1 l Wasser (12° dH) bei einer Temperatur von 30°C aufgelöst. Fünf Stücke gebleichte Baumwolle à je 10 g werden in diesem Bad bei 30°C während 15 Minuten gewaschen, dann unter kaltem, fliessendem Wasser gespült und in einer Schwingmaschine bei einer Tourenzahl von ca. 1000 Touren/Minute während 30 Sekunden zentrifugiert. Dieser Waschprozess wird 3 mal durchgeführt, dann werden die Baumwollstücke getrocknet und nach der Methode von Ganz mit einem Farbmessgerät (RFC 3 von Zeiss) auf ihren Weissgrad bestimmt. Es wird ein hoher Weissgrad, von über 175, erreicht.

Beispiel 11: Es wird analog wie in Beispiel 10 vorgegangen, wobei jedoch anstelle der Verbindung (102) die Verbindung (103) verwendet wird. Nach 3facher Wäsche wird ein hoher Weisseffekt erhalten.
Wird bei 60°C oder 90°C statt bei 30°C unter sonst gleichen Bedingungen gewaschen, liegen die resultierenden Weisseffekte noch höher.

Beispiel 12: Ein Waschpulver wird wie unter Beispiel 10 beschrieben hergestellt. Dann werden zu je 100 g dieses Granulates 3 g Diperoxydodecandisäure (DPDDA) im trockenen Zustand homogen zugemischt.
Lagertest Proben des so erhaltenen Waschmittels W (Granulat A + Persäure B) werden:

- einerseits zur Kontrolle des Ausgangswertes, sofort durch Extraktion und spektrophotometrische Extinktions-Messung auf den Gehalt an FWA (fluorescent whitening agent bzw. optischer Aufheller) bestimmt, (theoretisch: 0.1 % FWA bezogen auf das Gewicht des Granulats A)
- andererseits in Handels-Waschpulver gerechten Karton-Paketen, d.h. mit Beschichtung, unter ausgewählten und kontrollierten Temperatur- und Luftfeuchtigkeits-Bedingungen während bestimmten Zeit-Perioden gelagert. Nach der Lagerung wird von jedem Waschmittel sofort der Gehalt an FWA bestimmt. Der Unterschied zum Ausgangswert, in Prozent ausgedrückt, ist ein Mass für die Stabilität des FWA gegenüber dem entsprechenden Bleichmittel im Waschpulver.

Die oben erwähnte FWA-Bestimmung wird wie folgt durchgeführt:
Das Waschpulver wird durch Mahlen gut homogenisiert und 1 g davon wird mit 200 ml Lösungsmittel, bestehend aus 9 Teilen Dimethylsulfoxid und 1 Teil Wasser versetzt, und bei Raumtemperatur während 30 Minuten gerührt. Dann wird während 30 Minuten zentrifugiert. Von der so erhaltenen, klaren Lösung wird eine Probe mit einer Pipette in eine 1 cm Quarz-Küvette transferiert und deren Extinktion im UV-Bereich, beim Absorptionsmaximum gegen eine Standard-Lösung des jeweiligen FWA gemessen. Die Extinktion ist der FWA-Konzentration proportional.

Das Paket wird offen bei einer Temperatur von 30°C und einer relativen Luftfeuchtigkeit von 80-85 % während 8 Wochen gelagert, wie das im Haushalt, z.B. in einem Waschraum, vorkommen kann. Die quantitative Bestimmung des FWA zeigt eine ausgezeichnete Stabilität des Aufhellers.

Beispiel 13: Waschmittel werden wie im Beispiel 12 hergestellt. Die Pakete werden unter gleichen Bedingungen, jedoch geschlossen und während 6 Monaten gelagert, wie es in der Praxis zwischen Herstellung des Waschmittels und dem Gebrauch im Haushalt vorkommen kann. Auch hier weist die Verbindung (102) eine sehr gute Stabilität auf.

Beispiel 14: Ein Waschmittel wird wie unter Beispiel 10 beschrieben hergestellt. Dann werden 100 g dieses Granulates mit 14 g K-Monopersulfat und 0,4 mg $CuSO_4$ (wasserfrei) im trockenen Zustand homogen gemischt.
Nach 3 Monaten Lagerung im geschlossenen Paket bei Raumtemperatur (20-25°C) ist nur ein sehr geringer, bei praktischer Anwendung des Waschmittels nicht feststellbarer, Anteil der Verbindung (102) zerstört.

Beispiel 15: Waschmittel werden wie unter Beispiel 10 hergestellt und behandelt, wobei jedoch anstelle der Verbindung (102) jeweils 0,1 g der Verbindungen (103), oder (108) oder (113) eingesetzt werden.
Die Lagertests werden wie in den Beispielen 12 und 13 beschrieben durchgeführt. Die Verbindungen (103), (108) und (113) zeigen dabei auch ausgezeichnete Stabilitäten.

Beispiel 16: Waschmittel werden wie in Beispiel 14 beschrieben hergestellt und behandelt, wobei jedoch die Verbindung (102) durch jeweils 0,1 g der Verbindungen (103), oder (108) oder (113) ersetzt wird. Auch unter diesen Bedingungen weisen die Verbindungen sehr gute Stabilitäten auf.

**Patentansprüche**

1. Dibenzofuranylbiphenylverbindungen der Formel (I)

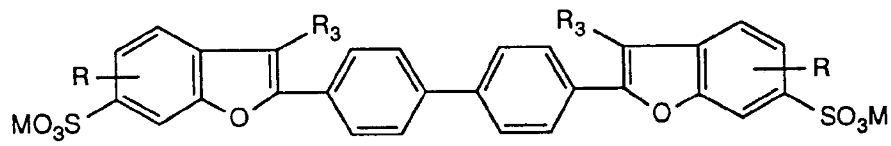

(I)

die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert sind und worin $R_3$ = $C_1$-$C_4$-Alkyl, Halogen oder Phenyl, M = Wasserstoff und/oder ein Aequivalent eines nicht-chromophoren Kations bedeuten.

2. Dibenzofuranylbiphenylverbindungen gemäss Anspruch 1 der Formel (II)

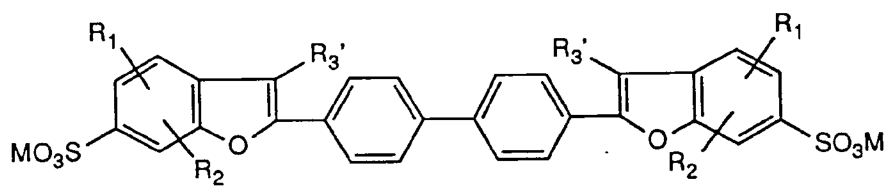

(II)

worin $R_1$ = Wasserstoff, $C_1$-$C_4$-Alkyl, Chlor, $C_1$-$C_4$-Alkoxy $R_2$ = Wasserstoff, $C_1$-$C_4$-Alkyl, Chlor oder $C_1$-$C_4$-Alkoxy, $R_3'$ = $C_1$-$C_4$-Alkyl, Chlor, Phenyl, M = Wasserstoff und/oder ein Aequivalent eines nicht chromophoren Kations bedeuten.

3. Dibenzofuranylbiphenylverbindungen gemäss Anspruch 2 der Formel (III)

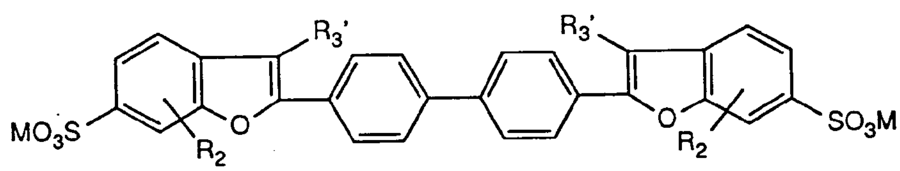

(III)

worin $R_2$ = Wasserstoff, $C_1$-$C_4$-Alkyl, Chlor, $C_1$-$C_4$-Alkoxy, $R_3'$ = $C_1$-$C_4$-Alkyl, Chlor oder Phenyl bedeuten und M die in Anspruch 2 angegebene Bedeutung hat.

4. Dibenzofuranylbiphenylverbindungen gemäss Anspruch 3 der Formel (IV)

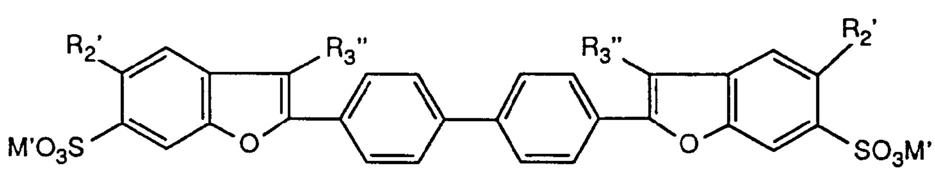

(IV)

worin $R_2'$ = Wasserstoff, Methyl, Ethyl, Chlor oder Methoxy, $R_3''$ = Methyl, Ethyl, Chlor oder Phenyl und M' = Wasserstoff oder Natrium bedeuten.

5. Verfahren zur Herstellung der Verbindungen der Formel (I) nach Anspruch 1, dadurch gekennzeichnet, dass man ein Mol der Verbindung der Formel (X)

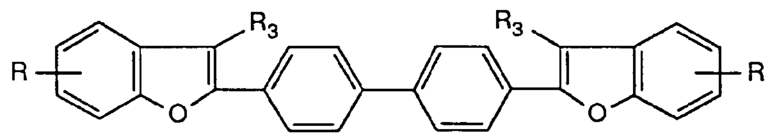 (X)

die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert ist und worin $R_3$ = $C_1$-$C_4$-Alkyl, Halogen oder Phenyl ist, mit

a) mindestens stöchiometrischen Mengen eines $SO_3$/Basen-Komplexes, in einem inerten organischen Lösungsmittel, bei Temperaturen von 20°C bis zum Siedepunkt des verwendeten Lösungsmittels umsetzt oder

b) die Verbindung der Formel (X) mit Chlorsulfonsäure bei 0-80°C oder

c) die Verbindung der Formel (X) mit Eisessig und konzentrierter Schwefelsäure oder Oleum bei Temperaturen von 80° bis 140°C

behandelt und die erhaltenen Sulfosäuren gegebenenfalls mit geeigneten Basen, wie wässrigen Alkali, Ammoniak oder Aminen neutralisiert.

**6.** Verwendung der in Anspruch 1 beanspruchten Verbindungen zum optischen Aufhellen von Textilmaterialien und Papier.

**7.** Verwendung der in Anspruch 1 beanspruchten Verbindungen in Hypochlorit enthaltenden Bleichmitteln sowie in Waschmitteln.

**8.** Lagerstabile Waschmittel enthaltend 0,5 bis 30% einer anorganischen oder organischen Persäure oder deren Salze oder Mischungen von Persäuren oder deren Salzen, sowie 0,03 bis 0,8% eines optischen Aufhellers oder einer Mischung optischer Aufheller, dadurch gekennzeichnet, dass die optischen Aufheller Bisbenzofuranylbiphenylverbindungen der Formel (I)

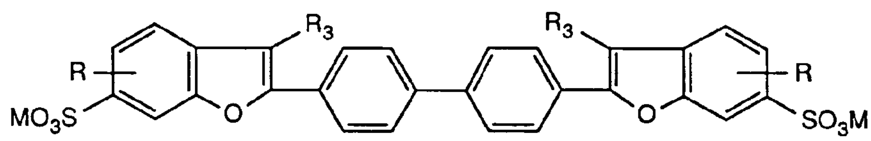 (I)

sind, die gegebenenfalls einfach oder mehrfach mit Resten R = Wasserstoff, $C_1$-$C_4$-Alkyl, $C_1$-$C_4$-Alkoxy, Halogen, Phenoxy und Benzyloxy substituiert sind und worin $R_3$ = $C_1$-$C_4$-Alkyl, Halogen oder Phenyl, M = Wasserstoff und/oder ein Aequivalent eines nicht-chromophoren Kations bedeuten.

**9.** Mittel nach Anspruch 8, das Persäuren oder deren Salze enthält, die bei einer Temperatur ab 20°C Textilmaterial bleichen.

**10.** Mittel nach Anspruch 9, das als Persäure bzw. -Salz, Diperoxydicarbonsäuren mit 6 bis 20 Kohlenstoffatomen, vorzugsweise mit 6-12 Kohlenstoffatomen, Persulfat und/oder Percarbonat gegebenenfalls in Kombination mit kleinen Mengen an Verbindungen, die die Bleichwirkung der Persäuren verstärken, enthält.

**11.** Mittel nach Anspruch 10, das als Persäure Diperoxydodecandisäure als organische Persäure enthält.

## Claims

**1.** A dibenzofuranylbiphenyl compound of the formula (I)

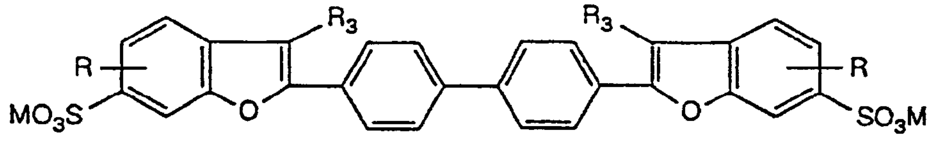 (I)

which is unsubstituted or is monosubstituted or polysubstituted by radicals R = hydrogen, $C_1$-$C_4$alkyl, $C_1$-$C_4$alkoxy, halogen, phenoxy and benzyloxy and in which $R_3$ is $C_1$-$C_4$alkyl, halogen or phenyl and M is hydrogen and/or one equivalent of a non-chromophoric cation.

**2.** A dibenzofuranylbiphenyl compound according to claim 1 of the formula (II)

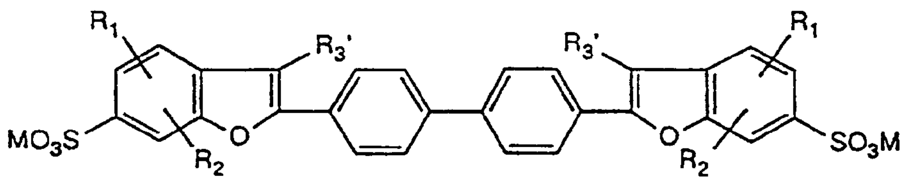

(II)

in which $R_1$ is hydrogen, $C_1$-$C_4$alkyl, chlorine or $C_1$-$C_4$alkoxy, $R_2$ is hydrogen, $C_1$-$C_4$alkyl, chlorine or $C_1$-$C_4$alkoxy, $R_3'$ is $C_1$-$C_4$alkyl, chlorine or phenyl, and M is hydrogen and/or one equivalent of a non-chromophoric cation.

**3.** A dibenzofuranylbiphenyl compound according to claim 2 of the formula (III)

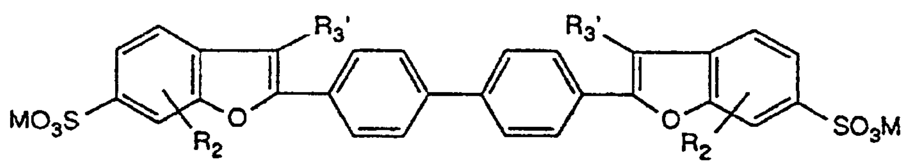

(III)

in which $R_2$ is hydrogen, $C_1$-$C_4$alkyl, chlorine or $C_1$-$C_4$alkoxy, $R_3'$ is $C_1$-$C_4$alkyl, chlorine or phenyl and M is as defined in claim 2.

**4.** A dibenzofuranylbiphenyl compound according to claim 3 of the formula (IV)

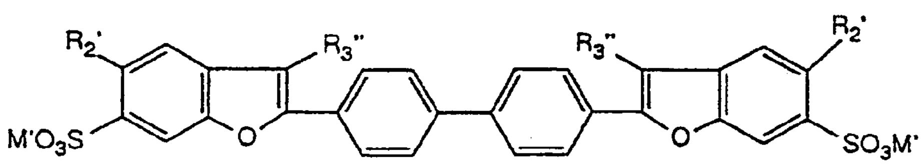

(IV)

in which $R_2'$ is hydrogen, methyl, ethyl, chlorine or methoxy, R3″ is methyl, ethyl, chlorine or phenyl and M' is hydrogen or sodium.

**5.** A process for the preparation of the compounds of the formula (I) according to claim 1, which comprises reacting one mole of the compound of the formula (X)

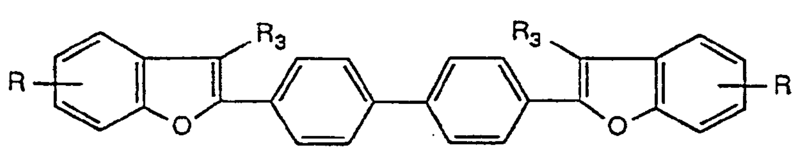

(X)

which is unsubstituted or is monosubstituted or polysubstituted by radicals R = hydrogen, $C_1$-$C_4$alkyl, $C_1$-$C_4$alkoxy, halogen, phenoxy and benzyloxy and in which $R_3$ is $C_1$-$C_4$alkyl, halogen or phenyl, with

a) at least stoichiometric amounts of an $SO_3$/base complex, in an inert organic solvent, at temperatures from 20°C up to the boiling point of the solvent used, or

b) treating the compound of the formula (X) with chlorosulfonic acid at 0-80°C or

c) treating the compound of the formula (X) with glacial acetic acid and concentrated sulfuric acid or oleum at temperatures from 80° to 140°C and neutralizing the resulting sulfonic acids, if appropriate,

with suitable bases, such as aqueous alkali, ammonia or amines.

6. The use of a compound claimed in claim 1 for the fluorescent whitening of textile materials and paper.

7. The use of a compound claimed in claim 1 in bleaching agents containing hypochlorite and also in detergents.

8. A detergent which is stable on storage and contains 0.5 to 30% of an inorganic or organic peracid or salts thereof or mixtures of peracids or salts thereof and also 0.03 to 0.8% of a fluorescent whitening agent or a mixture of fluorescent whitening agents, in which the fluorescent whitening agent is a bisbenzofuranylbiphenyl compound of the formula (I)

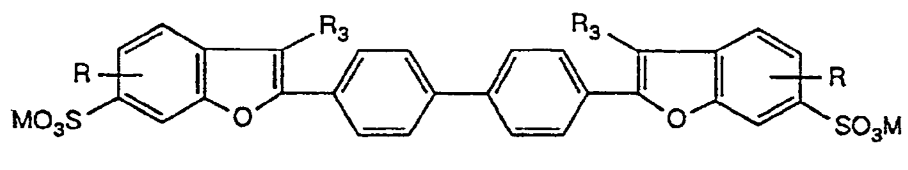

(I)

which is unsubstituted or is monosubstituted or polysubstituted by radicals R- hydrogen, $C_1$-$C_4$alkyl, $C_1$-$C_4$alkoxy, halogen, phenoxy and benzyloxy and in which $R_3$ is $C_1$-$C_4$alkyl, halogen or phenyl and M is hydrogen and/or one equivalent of a non-chromophoric cation.

9. An agent according to claim 8, which contains peracids or salts thereof which bleach textile materials at a temperature down to 20°C.

10. An agent according to claim 9, which contains, as the peracid or persalt, diperoxydicarboxylic acids having 6-20 carbon atoms, preferably 6-12 carbon atoms, persulfate and/or percarbonate, if appropriate in combination with small amounts of compounds which enhance the bleaching action of the peracids.

11. An agent according to claim 10 which contains, as the peracid, diperoxydodecanedioic acid as the organic peracid.

## Revendications

1. Composé du type di(benzofuranyl)biphényle de formule (I):

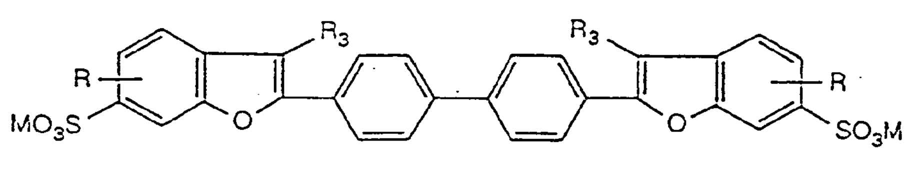

(I)

le cas échéant, mono- ou polysubstitué par des radicaux R représentant l'hydrogène, un radical alkyle en $C_1$-$C_4$ ou alcoxy en $C_1$-$C_4$, un halogène, le groupement phénoxy ou benzyloxy, dans laquelle :

$R_3$ représente un radical alkyle en $C_1$-$C_4$, un halogène ou le radical phényle, et

M représente l'hydrogène et/ou un équivalent d'un cation non chromophore.

2. Composé du type di(benzofuranyl)biphényle selon la revendication 1, de formule (II) :

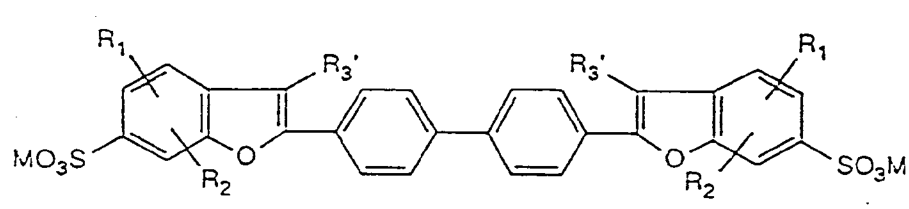

(II)

dans laquelle :

$R_1$ représente l'hydrogène, un radical alkyle en $C_1$-$C_4$, le chlore ou un radical alcoxy en $C_1$-$C_4$,
$R_2$ représente l'hydrogène, un radical alkyle en $C_1$-$C_4$, le chlore ou un radical alcoxy en $C_1$-$C_4$,
$R'_3$ représente un radical alkyle en $C_1$-$C_4$, le chlore ou le radical phényle, et
M représente l'hydrogène et/ou un équivalent d'un cation non chromophore.

3. Composé du type di(benzofuranyl)biphényle selon la revendication 2, de formule (III) :

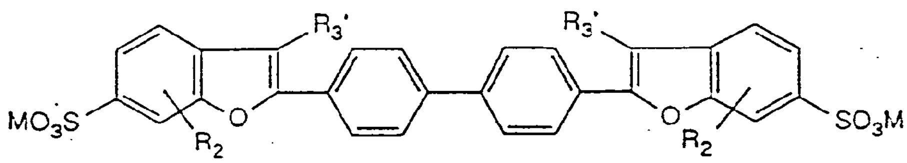
(III)

dans laquelle :

$R_2$ représente l'hydrogène, un radical alkyle en $C_1$-$C_4$, le chlore ou un radical alcoxy en $C_1$-$C_4$,
$R'_3$ représente un radical alkyle en $C_1$-$C_4$, le chlore ou le radical phényle, et
M a les mêmes significations que dans la revendication 2.

4. Composé du type di(benzofuranyl)biphényle selon la revendication 3, de formule (IV) :

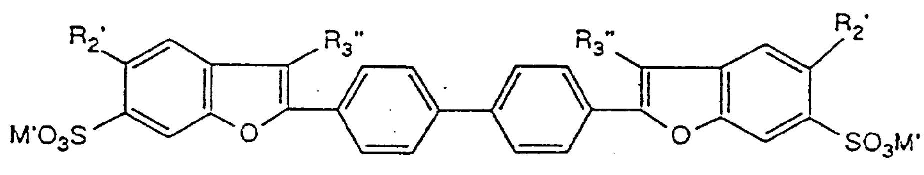
(IV)

dans laquelle :

$R'_2$ représente l'hydrogène, le radical méthyle ou éthyle, le chlore ou le radical méthoxy,
$R''_3$ représente le radical méthyle, éthyle ou phényle, ou le chlore, et
M′ représente l'hydrogène ou le sodium.

5. Procédé de préparation d'un composé de formule (I) selon la revendication 1, caractérisé en ce qu'une mole du composé de formule (X) :

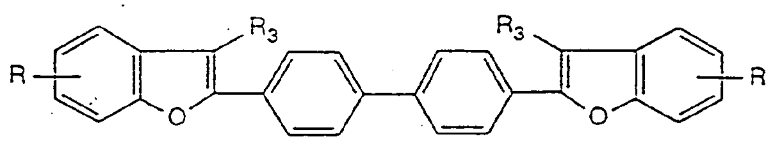
(X)

le cas échéant mono- ou polysubstitué par des radicaux R représentant l'hydrogène, un radical alkyle en $C_1$-$C_4$ ou alcoxy en $C_1$-$C_4$, un halogène, le radical phénoxy ou benzyloxy, et dans laquelle $R_3$ représente un radical alkyle en $C_1$-$C_4$, un halogène ou le radical phényle,

a) est mis à réagir avec une quantité stoechiométrique d'un complexe $SO_3$/bases dans un solvant organique inerte, à une température comprise entre 20°C et le point d'ébullition du solvant utilisé, ou le composé de formule (X) est traité avec
b) de la chlorhydrine sulfurique à des températures comprises entre 0 et 80°C, ou
c) de l'acide acétique glacial et de l'acide sulfurique concentré ou de l'huile à des températures comprises entre 80°et 140°C, puis, le cas échéant, les acides sulfoniques obtenus sont neutralisés à l'aide de bases appropriées, telles que des solutions aqueuses d'alcali, d'ammoniac ou d'amines.

6. Utilisation des composés de la revendication 1, pour l'azurage optique de matériaux textile et du papier.

7. Utilisation des composés selon la revendication 1, dans des produits de blanchiment contenant de l'hypochlorite tel que les détergents.

8. Détergent stable au stockage contenant 0,5 à 30% d'un peracide organique ou minéral ou ses sels ou un

mélange de peracides ou de leurs sels, 0,03 à 0,8% d'un azureur optique ou d'un mélange d'azureurs optiques, caractérisé en ce que les azureurs optiques sont des composés du type di(benzofuranyl)biphényle de formule (I) :

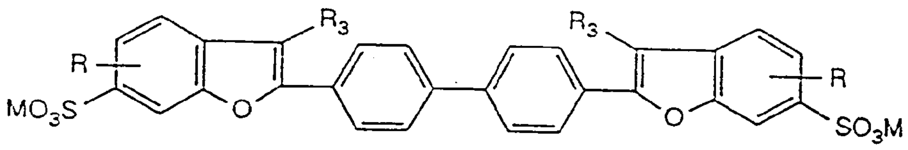 (I)

le cas échéant, mono- ou polysubstitué par des radicaux R représentant l'hydrogène, un radical alkyle en $C_1$-$C_4$ ou alcoxy en $C_1$-$C_4$, un halogène, le radical phénoxy ou benzyloxy, dans laquelle :

$R_3$ représente un radical alkyle en $C_1$-$C_4$, un halogène ou le radical phényle, et

M représente l'halogène et/ou un équivalent d'un cation non chromophore.

**9.** Détergent selon la revendication 8, contenant des peracides ou leurs sels, qui blanchit les matériaux textile à partir d'une température de 20°C.

**10.** Détergent selon la revendication 9, contenant comme peracides ou leurs sels, des acides diperoxydicarboxyliques contenant 6 à 20 atomes de carbone, de préférence 6 à 12 atomes de carbone, du persulfate et/ou du percarbonate, le cas échéant en combinaison avec une petite quantité de composés qui augmentent l'action de blanchiment des peracides.

**11.** Détergent selon la revendication 10, contenant à titre de peracides organiques, l'acide diperoxydodécanedioïque.